# EUROPÄISCHE PATENTSCHRIFT

(11) **EP 1 988 781 B1**
(45) Veröffentlichungstag und Bekanntmachung des Hinweises auf die Patenterteilung: **28.06.2017**
(21) Anmeldenummer: 07726448.9
(22) Anmeldetag: 20.02.2007
(51) Int. Cl.: A22C 7/00

(54) **VERFAHREN UND VORRICHTUNG ZUM HERSTELLEN VON GEFORMTEN FLEISCHPORTIONEN AUS GANZEN, GEWACHSENEN FLEISCHSTÜCKEN**
METHOD AND DEVICE FOR PRODUCING MOLDED PORTIONS OF MEAT FROM WHOLE CUTS OF NATURAL MEAT
PROCÉDÉ ET DISPOSITIF POUR PRODUIRE DES PORTIONS DE VIANDE FAÇONNÉES À PARTIR DE MORCEAUX DE VIANDE ENTIERS NON RECONSTITUÉS

(30) Priorität: 20.02.2006 DE 102006008132; 04.05.2006 DE 102006021139
(43) Veröffentlichungstag der Anmeldung: 12.11.2008
(73) Patentinhaber: Nienstedt GmbH, 45721 Haltern am See (DE)
(72) Erfinder: GRONEBERG-NIENSTEDT, Petra, 45721 Haltern am See (DE); GUTMANN, Michael, 45721 Haltern am See (DE)
(74) Vertreter: Bungartz, Klaus Peter
(86) Internationale Anmeldenummer: PCT/EP2007/051627
(87) Internationale Veröffentlichungsnummer: WO 2007/096363

(56) Entgegenhaltungen:
- EP-A- 1 470 754
- EP-A1- 0 288 592
- WO-A-2006/105821
- DE-A1- 10 164 637
- US-A1- 2005 282 482
- US-B1- 6 826 989

## Beschreibung

Die Erfindung betrifft ein Verfahren zum Herstellen von geformten Fleischportionen aus ganzen, gewachsenen Fleischstücken nach dem Oberbegriff des Anspruchs 1, das aus zwei getrennten Teilprozessen besteht, die nacheinander ausgeführt werden: Zum einen Fleischstücke kontrolliert und ohne einen notwendigen Formschnitt auf ein Zielgewicht bringen und zum anderen Fleischstücke kontrolliert in ein vorgegebenes, dreidimensionales Produkt überführen. Ferner betrifft die Erfindung eine Vorrichtung nach dem Oberbegriff des Anspruchs 12 zur Durchführung eines solchen Verfahrens.

Das Verfahren weist die Verfahrensschritte:
- Einbringen von Stücken ganzen, gewachsenen Fleischs in eine Verarbeitungslinie,
- Aussortieren derjenigen Fleischstücke, die ein Sollgewicht der Fleischportionen nicht erreichen oder Zusammenstellen von Clustern von Fleischstücken aus Einzelfleischstücken mit einem Bruchteil des Sollgewichtes,
- Beschneiden derjenigen Fleischstücke, deren Gewicht ein Sollgewicht der Fleischportionen überschreitet,
- Auswählen derjenigen Fleischstücke oder der Cluster von kleineren Fleischstücken, deren Gewicht einem Sollgewicht entspricht,
- Einfrieren der ausgewählten Fleischstücke zu gefrorenen Fleischportionen und
- Formen der gefrorenen Fleischportionen durch Einlegen in eine und Einpressen oder Einpressen und Stanzen in einer Formmulde zu den geformten Fleischportionen
auf.

Dieses Verfahren soll die Verarbeitung aller Arten gewachsenen Fleisches einschließen, insbesondere auch Rinder-, Schweine-, Schaf-, Lamm-, Wild-, Geflügel- oder Fischfleisch, einschließlich der jeweiligen Innereien.

In der Lebensmittelindustrie ist bekannt, gewachsene Fleischstücke in einem Schritt auf Gewicht und Form zu schneiden. Dies geschieht beispielsweise durch Schneiden mit Wasserstrahl oder konventionell mit Messern. Nachteilig ist dabei, dass eine große Menge an Abschnitten oder an nicht gewünschten Produkten entsteht, die im weiteren Prozess nicht verwendet werden können. Außerdem lässt sich das Höhenprofil des Produktes nur sehr eingeschränkt steuern.

Ein Verfahren der eingangs genannten Art ist aus der DE 101 64 637 A1 bekannt. Bei diesem Verfahren werden ganze gewachsene Fleischstücke sortiert und bei Bedarf zugeschnitten, damit die geforderten Portionen hergestellt werden können. Wird das Mindestgewicht nicht erreicht, werden mehrere kleinere Fleischstücke zusammen verarbeitet.

Dieses bekannte Verfahren hat jedoch den Nachteil, dass der Kunde häufig ein Stück Fleisch nachfragt, das nicht aus mehreren Teilen besteht, sondern aus einem einzigen Stück gewachsenen Fleisches. Dies hat seinen Grund zum einen in der vom späteren Endkunden geäußerten Vorstellung, dass das Fleisch "schnitzelartig" zusammenhängend sein soll, und zum anderen in der Gefahr, dass beim späteren Garen die Einzelteile wieder auseinander fallen.

Kann aber das zu kleine Teil nicht durch Hinzufügen eines anderen Teils an das Sollgewicht herangeführt werden, können nur noch diejenigen Teile verwendet werden, die von vorne herein entweder genau die geforderte Größe aufweisen oder die das Sollgewicht überschreiten, so dass durch Abschneiden eines Teils des gewachsenen Fleisches das Sollgewicht erreicht werden kann.

Ein weiterer Nachteil dieses Verfahrens besteht darin, dass die gewachsenen Fleischstücke auf Gewicht beschnitten werden, wobei die Größe des Stückes hierbei zunächst nur eine untergeordnete Rolle spielt. Ein sehr flaches Stück könnte somit die Gewichtskontrolle passieren und würde dann tiefgefroren, wobei dieses Stück dann für den Formgebungsprozess in der Formgebungsmaschine kaum geeignet wäre, weil es aufgrund seiner wohl zu großen Breite nicht in die Formmulde einpressbar ist, ohne dass ein Teil des Produktes abgeschert und damit nicht mehr weiterverarbeitet werden würde. Dies hätte zur Folge, dass die geforderte Menge des Fleisches im Endprodukt nicht mehr enthalten wäre, somit ein Qualitätsproblem auftreten würde.

Aus der DE 101 41 989 A1 ist ein Verfahren zum Ausstanzen einer in der Größe definierten Fleischportion aus einer plattenartig vorgeformten Masse bekannt. Bei diesem Verfahren werden die gewünschten Stücke schon in der später gewünschten Form, etwa einer runden Scheibe für einen speziellen Hamburger, aus dem plattenartigen Material herausgestanzt.

Der Nachteil dieses Verfahrens liegt darin, dass insbesondere bei kleineren Stücken die Platte des Materials naturbedingt sehr klein ist, so dass sich meist nur ein Stück aus dem Rohmaterial herausstanzen lässt, während der übrige Teil als nicht verwertbar einem anderen Verfahren zugeführt werden muss, obwohl an sich die Gesamt Menge des verarbeiteten Stückes genug Volumen für ein zweites Stück geliefert hätte.

Ein ähnliches Verfahren ist auch aus der DE 10 2005 016 159 A1 bekannt, bei der ebenfalls mehrere Teilstücke zu einem gemeinsamen Teil zugefügt werden, aus dem dann die einzelnen Formkörper ausgestanzt werden sollen.

Allgemein bekannt ist ferner ein Verfahren, bei dem das gewachsene Fleischstück in einem ersten Schritt zu einem ebenen Formstück gewalzt und in einem zweiten Schritt die gewünschte Form herausgeschnitten wird. Nachteilig bei diesem Verfahren ist die schlechte Steuerung der Form des Endprodukts, die geringe Möglichkeit, ein echtes dreidimensionales Produkt zu erhalten und der weiterhin hohe Anteil an im weiteren Prozess nicht verwertbaren Abschnitten.

Aufgabe der Erfindung ist es daher, ein Verfahren zum Herstellen von geformten Fleischportionen aus ganzen, gewachsenen Fleischstücken zu schaffen, bei dem die geformte Fleischportion in zwei Schritten hergestellt wird, so dass sie ein vorgegebenes Gewicht und eine vorgegebene Form erhält, ohne dass unnötiger Verschnitt entsteht. Ferner ist es Aufgabe der Erfindung, das vorgegebene Gewicht so zu erreichen, dass minimale Restriktionen hinsichtlich der gewünschten Fleischportionen entstehen, und größtmögliche Flexibilität in der Gestaltung der vorgegebenen dreidimensionalen Form der Fleischportion zu erhalten. Eine weitere Aufgabe besteht darin, eine Vorrichtung zu schaffen, mit der ein solches Verfahren durchgeführt werden kann.

Diese Aufgabe wird nach der Erfindung bezüglich des Verfahrens durch ein Verfahren nach Anspruch 1 gelöst. Bezüglich der Vorrichtung wird die Aufgabe durch eine Vorrichtung nach Anspruch 12 gelöst.

Erfindungsgemäß werden die Fleischstücke nach dem Bringen auf ein vorgegebenes Gewicht eingefroren und dann umgeformt. Zunächst werden die Fleischstücke gewogen und sortiert. Dies kann manuell oder maschinell geschehen, wobei bei manueller Wägung anschließend gleich der Zuschnitt erfolgen kann. Beim automatischen Verfahren werden die Fleischstücke beispielsweise über ein Förderband zugeführt, das eine integrierte Waage aufweist. Über aus der Logistik bekannte Schwenkarme wird in Anhängigkeit vom Ergebnis des Wiegens das betroffene Stück kontrolliert vom Förderband abgeführt oder durchgelassen. Fleischstücke mit zu geringem Gewicht werden dann aussortiert und einem anderen Verarbeitungsverfahren zugeführt oder zu Clustern zusammengefasst, die wiederum als Fleischstück dem hier beschriebenen Verfahren zugeführt werden können.

Die Stücke, die das Sollgewicht überschreiten, werden auf dieses Sollgewicht zugeschnitten, um für alle verarbeiteten Stücke die geforderte Fleischmasse zu verarbeiten. Dieser Schritt wird als Portionssteuerung bezeichnet, es werden also die Fleischstücke portionsgerecht verarbeitet, wobei das Gewicht das entscheidende Kriterium für den Eingang des Stückes in den Verarbeitungsprozess darstellt. Hierbei wird jedoch kein Formschnitt durchgeführt, so dass Verschnitte durch die notwendige Formgebung vermieden oder minimiert werden können.

Das Zuschneiden kann mit Hilfe von Messern manuell oder automatisch, im letzteren Fall konventionell oder über Ultraschallanregung betrieben, mittels Wasserstrahl oder mit alternativen Schneidmethoden, wie z.B. Laserschneiden, erfolgen. Ist einmal das Stück gewogen, kann die geforderte Schnittlinie, um dieses "übergewichtige" Stück auf das Sollgewicht zu reduzieren, über eine optische Volumenerkennung und eine Gewichtsbestimmung anhand der Dichte des Fleisches von einem Computer errechnet werden. Dabei kann die Schnittlinie vorteilhaft so berechnet werden, dass der Anteil an nicht im Prozess verwendbaren Abschnitten minimiert wird. Von Vorteil ist, dass bei Berechnung der Schnittlinie praktisch keine Rücksicht auf die gewünschte Endform des Produktes genommen werden muss.

Sind die Stücke alle in Hinblick auf das Sollgewicht vereinheitlich, werden sie, sofern dies nicht schon vorher geschehen und gewünscht ist, gewürzt und für den späteren Garprozess präpariert. Hierzu wird bevorzugt eine Marinade verwendet und das Fleisch in einem Tumbler verarbeitet. Dieses Verfahren ist allgemein bekannt und führt zum Einmassieren der Marinade sowie zum Austritt von Proteinen aus dem Fleisch. Alternativ kann auch eine Würzflüssigkeit injiziert werden, die über eine Vielzahl von Injektionsnadeln in das Fleisch eingebracht wird und somit schneller und wirksamer zur Verfügung steht, während die Verweildauer in einem Tumbler in der Regel zumindest 15 Minuten bis hin zu einer Stunde oder mehr beträgt.

Ein besonderer Vorzug des erfindungsgemäßen Verfahrens besteht darin, dass das Marinieren nun mit Fleischstücken durchgeführt werden kann, deren Fleischmasse der gewünschten Masse entspricht und dass Abschnitte, die hierfür nicht benötigt werden, ohne Marinade zurückgeführt oder einem anderen Verfahren zugeführt werden können. Dies ist vorteilhaft, weil Abnehmer üblicherweise das Marinieren des Fleisches mit einer eigenen Marinade wünschen, so dass Abschnitte marinierten oder getumbelten Fleisches nur für diesen Abnehmer wiederverwendet werden könnten, oder sogar, falls dieser Abnehmer keine Cluster von Einzelstücken oder Stücke in der Größe des Abschnittes zulässt, gar nicht weiter verwendet werden können.

Die wie oben beschrieben präparierten Fleischstücke werden nun während des Einfrierens konturgerecht vorgeformt. Besonders effizient geschieht dies dadurch, dass das Fleisch auf ein Transportband aufgebracht wird, in dem sich Gefrierformen befinden, in die jeweils eine Portion eingebracht wird. Die Portion kann dabei entweder über eine maschinelle Drückvorrichtung oder manuell in die Einsenkung eingebracht werden. Auch ein Ansaugen über ein Vakuum ist möglich.

Ist das ausgewählte Fleischstück sehr dünn und breit, kann auch ein trichterartiger Einlauf vorgesehen sein, über den das Fleisch entweder selbsttätig oder per Druck in die Gefrierform gelangt. Auch eine besondere Mechanik zum Falten eines dünnen Abschnittes könnte vorgesehen sein. Diese kann zum Beispiel selbsttätig überhängende Bereich erkennen und umlegen, so dass sie in die Gefrierform fallen.

Die Gefriermulden können vorteilhaft so gefertigt sein, dass ein Festfrieren während des Gefrierens der Fleischstücke an der Gefriermulde verhindert oder ein Entformen festgefrorener Fleischstücke erleichtert bzw. ermöglicht wird. Hierzu kann die Gefriermulde zweiteilig und/oder flexibel ausgebildet sein oder mit einer zum Beispiel hydrophoben Beschichtung versehen sein. Die Beschichtung kann zum Beispiel aus einem Öl bestehen und/oder auch eine feste Beschichtung sein, die etwa aus einem Kunststoff, beispielsweise Polytetraflourethylen, bestehen kann. Alternativ oder zusätzlich zu den beschriebenen Maßnahmen können die Gefriermulden später auch über eine externe Wärmezufuhr, entweder eine Heizung oder ein Warmwasserbad, beheizt werden, so dass die Randbereiche zum leichteren Entnehmen des gefrorenen Fleisches leichtantauen.

Nach dem Entformen werden die Fleischstücke der Gefrierstation zugeführt, die auf übliche Weise das Fleisch tief gefriert. Dies kann zum Beispiel durch einen Spiralfroster oder einen Tunnelfroster geschehen. Die Temperatur beträgt dabei beispielsweise zwischen -6 und -16°C.

Nach dem Tieffrieren werden die einzelnen Stücke bei einer bevorzugten Ausgestaltung des Verfahrens aus der Gefriermulde entnommen und einem so genannten "Shaper" zugeführt, der dann die Fleischstücke in die gewünschte Endform presst. Dabei können die gewichtskontrollierten Fleischstücke nach dem aus der DE 101 64 637 A1 bekannten Verfahren so umgeformt werden, dass aus jeweils einer Fleischportion ein Produkt mit vorbestimmter dreidimensionaler Form entsteht ("Standardisierung"). Alternativ kann jeweils eine Fleischportion nach dem aus der DE 10 2005 016 159 A1 bekannten Verfahren in mehrere Produkte mit vorbestimmter dreidimensionaler Form überführt werden ("Standard-Teilung").

Wichtig für die erfindungsgemäße Optimierung des Verfahrens durch die Erfindung ist dabei, dass der zuvor gefrorene Fleischkörper durch den Zwischenschritt des Vorformens eine Form aufweist, die der Grundfläche der Formmulde des Shapers entspricht oder kleiner ist. So kann vermieden werden, dass der Stempel des Shapers einen Teil des Fleisches abkantet, das dann nicht mehr in die Formmulde fallen würde. Zusätzlich werden die Verformungsgrade des Fleisches im gefrorenen Zustand reduziert, was zu einer schonenderen Verarbeitung der Fleischportion beiträgt.

Schließlich kann über die vorweg genommene Vorformung auch dem späteren Endprodukt Rechnung getragen werden, indem das Vorformen so erfolgt, dass es nicht notwendig ist, eine extreme Formgebung durch alleiniges Verdrängen des Materials nur im Shaper zu realisieren. Neben einer das Fleisch schonenden Umformung bedeutet dies auch, dass eine höhere Flexibilität bei der Gestaltung der Formmulde möglich ist und dass die Taktzeiten aufgrund des geringeren erforderlichen Verformungsgrades reduziert werden können. Anschließend können die geformten Fleischprodukte jeder Art der lebensmitteltechnischen Verarbeitung zugeführt werden, insbesondere können sie mit einer Panade versehen und einem Garprozess durch Braten, Frittieren, Dünsten oder Kochen unterzogen werden.

Durch das erfindungsgemäße Verfahren kann nun unabhängig von der Dicke des Fleischprodukts eine gleiche Größe bei gleichem Gewicht für eine Vielzahl von Produkten aus unterschiedlichstem Ausgangsmaterial erzielt werden. Es ist somit sowohl eine Steuerung der Portionsgröße als auch der-form möglich.

Die Erfindung betrifft darüber hinaus auch eine Vorrichtung zur Umsetzung dieses Verfahrens.

Weitere Merkmale und Vorteile der Erfindung ergeben sich aus den Unteransprüchen und aus der nachfolgenden Beschreibung eines bevorzugten Ausführungsbeispiels.

Das bevorzugte Ausführungsbeispiel des Verfahrens zum Herstellen von gewichtsgenauen geformten Fleischportionen mit vorgegebener dreidimensionaler Form erfolgt so, dass gewachsene Fleischstücke, die nicht notwendig das Zielgewicht und/oder die Zielform aufweisen, in die Verarbeitungslinie eintreten und zunächst gewogen werden. Passt zufällig das Gewicht des gewachsenen Stückes mit dem Zielgewicht zusammen, wird dieses Stück weitergeleitet.

Wahrscheinlicher ist jedoch der Fall, dass das Stück entweder zu klein oder zu groß ist. Im erstgenannten Fall wird es aussortiert und einer anderen Verwendung zugeführt. Im letztgenannten Fall kann durch Abschneiden eines Teils das Sollgewicht erreicht werden. Hierzu wird das Volumen des Stückes optisch erfasst, über die Dichte die notwendige Schnittlinie berechnet und dann das Stück in einen oder mehrere Abschnitte und ein für das Verfahren geeignetes Stück geteilt.

Die so ausgewählten Stücke, die dem Sollgewicht entsprechen, werden dann gewürzt und getumbelt. Dann erfolgt der Schritt der Formanpassung. In einem ersten Schritt wird das Fleisch dabei in eine Gefrierform eingebracht und gefroren. Hierbei erfolgt eine erste Vorformung.

In einem zweiten Schritt wird dann das gefrorene Fleisch aus dieser Form wieder entnommen und in einem Shaper endgültig in die gewünschte dreidimensionale Form gebracht. Hierzu wird das Fleisch in eine Formmulde des Shapers eingelegt, deren Grundfläche etwas größer als die der Gefrierform ist. Dies stellt sicher, dass das Fleisch während des anschließenden Pressvorgangs sicher in der Form verbleibt und sich wie gewünscht verteilt, ohne seitlich aus der Form herauszuquellen.

Der so hergestellte, gefrorene Portionskörper kann dann auf übliche Weise weiter zerteilt oder anderweitig weiterverarbeitet, etwa paniert oder gegart werden.

## Patentansprüche

1. Verfahren zum Herstellen von geformten Fleischportionen aus ganzen, gewachsenen Fleischstücken, insbesondere aus Rinder-, Schweine-, Schaf-, Lamm-, Wild-, Geflügel- oder Fischfleisch aller Art, mit den Verfahrensschritten:
• Einbringen der Stücke ganzen gewachsenen Fleischs in eine Verarbeitungslinie,
• Aussortieren derjenigen Fleischstücke, die ein Sollgewicht der Fleischportionen nicht erreichen,
• Beschneiden derjenigen Fleischstücke, deren Gewicht ein Sollgewicht der Fleischportionen überschreitet,
• Auswählen derjenigen Fleischstücke deren Gewicht einem Sollgewicht entspricht, als ausgewählte Fleischstücke, die der weiteren Bearbeitung zugeführt werden,
• Vorformen der ausgewählten Fleischstücke,
• Einfrieren der vorgeformten ausgewählten Fleischstücke zu gefrorenen Fleischportionen,
• Formen der gefrorenen Fleischportionen durch Einlegen und anschließendes Einpressen in eine Formmulde zu den geformten Fleischportionen mit vorgegebenem Gewicht und vorgegebener dreidimensionaler Form,
**dadurch gekennzeichnet, dass**
die ausgewählten Fleischstücke zur Durchführung des Vorformens unter Vorformung in eine Gefrierform eingebracht werden, nach dem Einfrieren aus der Gefrierform entnommen und anschließend in die Formmulde zum Einpressen eingelegt werden, wobei die ausgewählten Fleischstücke durch die Vorformung in eine Form gebracht werden, deren Grundfläche der Grundfläche der Formmulde entspricht oder deren Grundfläche kleiner als die Grundfläche der Formmulde ist.

2. Verfahren zum Herstellen von geformten Fleischportionen nach Anspruch 1, **dadurch gekennzeichnet, dass** mehrere Verfahren mit unterschiedlichen Sollgewichten durchgeführt werden und vor dem Beschneiden derjenigen Fleischstücke, deren Gewicht ein Sollgewicht der Fleischportionen überschreitet, diese Fleischstücke nach der Möglichkeit sortiert werden, möglichst ganzzahlige Vielfache von Sollgewichten hieraus zuzuschneiden, und diese Fleischstücke dann dem jeweiligen Verfahren zugeführt werden.

3. Verfahren zum Herstellen von geformten Fleischportionen nach einem der beiden vorhergehenden Ansprüche, **dadurch gekennzeichnet, dass** die Fleischstücke, deren Gewicht ein Sollgewicht der Fleischportionen deutlich überschreitet, zumindest dann, wenn ein absehbarer Abschnitt dieses Fleischstücke das Sollgewicht erneut überschreiten wird und hieraus somit ein weiteres Fleischstück herstellbar ist, derart geteilt werden, dass die Fleischportion und der Abschnitt der Form der Formmulde möglichst nahe kommen.

4. Verfahren zum Herstellen von geformten Fleischportionen nach einem der vorhergehenden Ansprüche, **dadurch gekennzeichnet, dass** die gewachsenen Fleischstücke vor dem Zuschneiden durch Walzen oder Pressen in eine flache Form vorgeformt werden.

5. Verfahren zum Herstellen von geformten Fleischportionen nach einem der vorhergehenden Ansprüche, **dadurch gekennzeichnet, dass** die ausgewählten Fleischstücke in eine Gefrierform eingelegt oder eingepresst werden, deren Grundfläche und Höhe geringer ist als die Grundfläche und Höhe der Formmulde, wobei zu große, flache Fleischstücke bei Einlegen in die Gefrierform durch Übereinanderbringen von Bereichen der Fleischstücke (z.B. Falten, Rollen) in die Gefrierform passend gemacht werden.

6. Verfahren zum Herstellen von geformten Fleischportionen nach einem der vorhergehenden Ansprüche, **dadurch gekennzeichnet, dass** die ausgewählten Fleischstücke vor dem Gefrieren gewürzt oder mariniert werden und/oder vor dem Gefrieren einem Tumbler oder Injektor zugeführt werden.

7. Verfahren zum Herstellen von geformten Fleischportionen nach einem der vorhergehenden Ansprüche, **dadurch gekennzeichnet, dass** die aus den ausgewählten Fleischstücken vorgeformten, gefrorenen Fleischportionen aus der Gefrierform entnommen werden, anschließend separiert werden und einer Formvorrichtung mit einer Mehrzahl von Formmulden zugeführt werden, wobei zum leichteren Entnehmen der gefrorenen Fleischportionen aus der Gefrierform diese zuvor über eine Heizung oder ein Warmwasserbad angewärmt werden können.

8. Verfahren zum Herstellen von geformten Fleischportionen nach einem der vorhergehenden Ansprüche, **dadurch gekennzeichnet, dass** als Gefriermulden Mulden in einem Transportband verwendet werden, über das die ausgewählten Fleischstücke einer Gefrierstation zugeführt werden.

9. Verfahren zum Herstellen von geformten Fleischportionen nach einem der vorhergehenden Ansprüche, **dadurch gekennzeichnet, dass** in der Formvorrichtung mit einer Mehrzahl von Formmulden in jede Formmulde eine gefrorene Fleischportion eingelegt wird und anschließend durch Pressen in jeder Formmulde eine geformte Fleischportion hergestellt wird oder durch Pressen und Stanzen in jeder Formmulde mehrere geformte Fleischportionen hergestellt werden.

10. Verfahren zum Herstellen von geformten Fleischportionen nach einem der vorhergehenden Ansprüche, **dadurch gekennzeichnet, dass** zur Erzielung einer unterschiedlichen Form der geformten Fleischportionen nach einem Garprozess die ausgewählten Fleischportionen unterschiedlich stark umgeformt werden, wobei hierzu gezielt die Fleischstücke so zugeschnitten werden, dass unterschiedliche Verformungsgrade in der Formmulde auftreten.

11. Verfahren zum Herstellen von geformten Fleischportionen nach einem der vorhergehenden Ansprüche, **dadurch gekennzeichnet, dass** die geformten Fleischportionen nach dem Formen paniert werden und/oder nochmals gewürzt werden und/oder nach eventuellen weiteren Verfahrensschritte, insbesondere durch Kochen, Dünsten oder Braten gegart werden.

12. Vorrichtung zur Herstellung von geformten Fleischportionen mit
• einer Sortiervorrichtung zum Sortieren des Fleisches in Stücke, deren Gewicht unterhalb eines Sollgewichtes liegt, Stücke, deren Gewicht einem Sollgewicht entspricht, und Stücke, deren Gewicht oberhalb eines Sollgewichtes liegt,
• einer Zuschneidevorrichtung, die übergewichtige Fleischstücke auf ein Sollgewicht zuzuschneiden vermag, wobei die gewichtsgenauen und zugeschnitten Fleischstücke oder gewichtsgenaue Cluster von Fleischstücken als ausgewählte Fleischstücke weiterverarbeitet werden,
• einer Transportvorrichtung zum Weitertransport der ausgewählten Fleischstücke,
• einer Gefrieranlage, durch die die Transportvorrichtung die ausgewählten Fleischstücke hindurchzuführen vermag und die die ausgewählten Fleischstücke einzufrieren vermag,
• einer Formvorrichtung, die die eingefrorenen ausgewählten Fleischstücke auf gesteuerte Weise in eine Form zu pressen und somit in eine Endform oder mehrere Endformen als Fleischportionen mit vorbestimmtem Gewicht und vorbestimmter dreidimensionaler Form zu überführen vermag,
**dadurch gekennzeichnet, dass**
die Vorrichtung zur Durchführung eines Verfahrens zum Herstellen von geformten Fleischportionen nach einem der vorhergehenden Ansprüche eingerichtet ist, wobei die Vorrichtung zur Durchführung des Schrittes des Vorformens Mittel zur Vorformung der ausgewählten Fleischstücke vor dem Gefrieren aufweist, in die ausgewählten Fleischstücke unter Vorformung einleg- oder eindrückbar sind und über die die vorgeformten, ausgewählten Fleischstücke gefrierbar sind.

13. Vorrichtung zur Herstellung von geformten Fleischportionen nach dem vorhergehenden Anspruch, **dadurch gekennzeichnet, dass** das Mittel zur Vorformung Gefriermulden aufweist, die Teil der Transportvorrichtung, insbesondere eingeprägte Formnester in einem Transportband, sind.

14. Vorrichtung zur Herstellung von geformten Fleischportionen nach einem der beiden vorhergehenden Ansprüche, **dadurch gekennzeichnet, dass** sie ein Mittel zur Gewichtsbestimmung der Fleischstücke aufweist, wobei diese das Gewicht insbesondere aufgrund eines erfassten Volumens und eines vorgegebenen Dichtewertes zu berechnen vermag.

## Claims

1. Method for producing shaped meat portions from pieces of whole mature meat, in particular from beef, pork, mutton, lamb, game meat, poultry meat or fish of all kinds, with the following method steps:
• Introducing the pieces of whole mature meat into a processing line,
• Sorting out those pieces of meat which do not reach a desired weight of the meat portions,
• Trimming those pieces of meat whose weight exceeds a desired weight of the meat portions,
• Selecting those pieces of meat whose weight corresponds to a desired weight as selected pieces of meat which are supplied for further processing,
• Pre-shaping the selected pieces of meat,
• Freezing the shaped selected pieces of meat into frozen meat portions,
• Shaping the frozen meat portions by placing them in a shaping mould in which they are then pressed into the shaped meat portions of a predefined weight and a predefined three-dimensional shape,
**characterised in that,**
in order to carry out the pre-shaping, the selected pieces of meat are inserted into a freezing mould for pre-shaping, removed from the freezing mould after freezing and then placed in the shaping mould for pressing, wherein the selected pieces of meat are made into a shape by the pre-shaping whose base area corresponds to the base area of the shaping mould or whose base area is smaller than the base area of the shaping mould.

2. Method for producing shaped meat portions in accordance with claim 1, **characterised in that** a plurality of methods are performed with different desired weights and, before trimming those pieces of meat whose weight exceeds a desired weight of the meat portions, these pieces of meat are sorted so as to cut as many whole multiples of desired weights from them as possible, and these pieces of meat are then supplied to the appropriate process.

3. Method for producing shaped meat portions in accordance with either one of the two preceding claims, **characterised in that** the pieces of meat, whose weight significantly exceeds a desired weight of the meat portions, at least if a foreseeable section of this piece of meat will again exceed the desired weight and it is then possible to produce another piece of meat from it, are divided in such a way that the meat portion and the section come as close as possible to matching the shape of the shaping mould.

4. Method for producing shaped meat portions in accordance with any one of the preceding claims, **characterised in that** the pieces of mature meat are pre-shaped by rolling or pressing in a shallow mould before cutting to size.

5. Method for producing shaped meat portions in accordance with any one of the preceding claims, **characterised in that** the selected pieces of meat are inserted or pressed into a freezing mould whose base area and height are smaller than the base area and height of the shaping mould, wherein flat pieces of meat that are too large when placed in the freezing mould are made to fit into the freezing mould by overlapping areas of the pieces of meat (e.g. folding, rolling).

6. Method for producing shaped meat portions in accordance with any one of the preceding claims, **characterised in that**, before freezing, the selected pieces of meat are seasoned or marinated and/or are fed into a tumbler or injector before freezing.

7. Method for producing shaped meat portions in accordance with any one of the preceding claims, **characterised in that** the frozen meat portions pre-shaped from the selected pieces of meat are removed from the freezing mould, then separated and supplied to a shaping device with a plurality of shaping moulds, wherein, in order to be able to remove the frozen meat portions more easily from the freezing mould, they may be heated beforehand by means of a heater or a warm water bath.

8. Method for producing shaped meat portions in accordance with any one of the preceding claims, **characterised in that** moulds can be used as freezing moulds on a conveyor belt by means of which the selected pieces of meat are supplied to a freezing station.

9. Method for producing shaped meat portions in accordance with any one of the preceding claims, **characterised in that**, in the shaping device with a plurality of shaping moulds, a frozen meat portion is placed in each shaping mould and a shaped meat portion is then produced by pressing in each shaping mould or several shaped meat portions are produced by pressing and stamping in each shaping mould.

10. Method for producing shaped meat portions in accordance with any one of the preceding claims, **characterised in that**, in order to achieve a different shape of the meat portions after a cooking process, the selected meat portions are produced in markedly different shapes wherein, in order to achieve this intentionally, the piece of meat are cut so that different degrees of deformation occur in the shaping mould.

11. Method for producing shaped meat portions in accordance with any one of the preceding claims, **characterised in that** the shaped meat portions are coated with breadcrumbs after the shaping and/or are again seasoned and/or are cooked in possible further method steps, in particular by boiling, steaming or roasting.

12. Device for producing shaped meat portions with
• a sorting device for sorting the meat into pieces, whose weight falls below a desired weight, pieces whose weight corresponds to a desired weight and pieces whose weight exceeds a desired weight,
• a cutting device which is able to cut the overweight pieces of meat to a desired weight, wherein the cut pieces of meat of the correct weight or clusters of pieces of meat of the correct weight are processed further as selected pieces of meat,
• a conveyor device for conveying the selected pieces of meat,
• a freezing system through which the conveyor device is able to transport the selected pieces of meat and which is able to freeze the selected pieces of meat,
• a shaping device which is able to press the frozen selected pieces of meat into a shape and, hence, is able to convert them into a final shape or several final shapes as meat portions of a predefined weight and predefined three-dimensional shape,
**characterised in that**
the device for performing a method for producing shaped meat portions according to any one of the preceding claims is configured wherein the device to perform the steps in pre-shaping has means to pre-shape the selected pieces of meat before freezing in which selected pieces of meat can be inserted or pressed during pre-shaping and by means of which the pre-shaped selected pieces of meat can be frozen.

13. Device for producing shaped meat portions according to the preceding claim, **characterised in that** the means for pre-shaping has freezing moulds which are part of the conveyor device, in particular impressed mould cavities in a conveyor belt.

14. Device for producing shaped meat portions according to the either one of the two preceding claims, **characterised in that** it has means for determining the weight of the pieces of meat wherein they are able to calculate the weight based in particular on a recorded volume and a predefined density value.

## Revendications

1. Procédé pour produire des portions de viandes façonnées à partir de morceaux de viandes entiers non reconstitués, notamment de viande de boeuf, de porc, de mouton, d'agneau, de gibier, de volaille ou de poisson de tout type comprenant les étapes suivantes :
• Introduire les morceaux de viande entiers non reconstitués dans une ligne de traitement,
• Extraire les morceaux de viande dont le poids est inférieur à un poids théorique,
• Couper les morceaux de viande dont le poids est supérieur à un poids théorique,
• Sélectionner les morceaux de viande dont le poids correspond à un poids théorique comme morceaux de viande sélectionnés à introduire dans la ligne de traitement,
• Préformer les morceaux de viande sélectionnés,
• Congeler les morceaux de viande préformés sélectionnés pour façonner des portions de viande congelées,
• Façonner les portions de viande congelées en introduisant les morceaux de viande dans une cavité de façonnage et en appliquant une pression pour former des portions de viande avec un poids et une forme tridimensionnelle prédéterminés,
**caractérisé en ce que**
les morceaux de viande sélectionnés pour la réalisation de la préformation sont introduits par préformation dans un moule de congélation, ces derniers sont prélevés du moule de congélation après la congélation, puis sont introduits dans la cavité de façonnage pour la pression, les morceaux de viande sélectionnés étant introduits par préformation dans un moule dont la surface de base correspond à la surface de base de la cavité de façonnage ou dont la surface de base est inférieure à la surface de base de la cavité de façonnage.

2. Procédé pour produire des portions de viande façonnées selon la revendication 1, **caractérisé en ce que** plusieurs procédés sont réalisés avec différents poids théoriques et que ces morceaux de viande, avant la coupe des morceaux de viande dont le poids est supérieur à un poids théorique des portions de viande, sont triés si possible de façon à pouvoir couper des portions de viande à poids théorique en multiples entiers et que ces morceaux de viande ensuite sont introduits dans les lignes de procédé respectives.

3. Procédé pour produire des portions de viande façonnées selon l'une quelconque des deux revendications précédentes, **caractérisé en ce que** les morceaux de viande dont le poids est largement supérieur au poids théorique des portions de viande, au moins lorsqu'une section prévisible de ces morceaux de viande présentera à nouveau un poids supérieur au poids théorique et que de cette façon un autre morceau de viande peut être produit, sont coupés de sorte que la portion de viande et la section se rapprochent le plus possible de la forme de la cavité de façonnage.

4. Procédé pour produire des portions de viande façonnées selon l'une quelconque des revendications précédentes, **caractérisé en ce que** les morceaux de viande non reconstitués avant la coupe sont préformés par laminage ou pression dans un moule plat.

5. Procédé pour produire des portions de viande façonnées selon l'une quelconque des revendications précédentes, **caractérisé en ce que** les morceaux de viande sélectionnés sont introduits ou pressés dans un moule de congélation dont la surface de base et la hauteur sont inférieures à la surface de base et la hauteur de la cavité de façonnage, des morceaux de viande plats et trop grands étant adaptés lors de l'introduction dans le moule de congélation par superposition des parties des morceaux de viande (p.ex. pliage, roulement) dans le moule de congélation.

6. Procédé pour produire des portions de viande façonnées selon l'une quelconque des revendications précédentes, **caractérisé en ce que** les morceaux de viande sélectionnés sont assaisonnés ou marinés avant la congélation et/ou sont amenés à un séchoir ou injecteur avant la congélation.

7. Procédé pour produire des portions de viande façonnées selon l'une quelconque des revendications précédentes, **caractérisé en ce que** les portions de viande congelées préformées à partir des morceaux de viande sélectionnés sont prélevées du moule de congélation, puis sont séparées et sont amenées à un dispositif de façonnage présentant une multitude de cavités de façonnage, les portions de viandes congelées pouvant être chauffées par un chauffage ou un bain d'eau chaude pour faciliter leur prélèvement du moule de congélation.

8. Procédé pour produire des portions de viande façonnées selon l'une quelconque des revendications précédentes, **caractérisé en ce que** des moules sont utilisés comme des moules de congélation dans un convoyeur par lequel les morceaux de viande sélectionnés sont transportés à une station de congélation.

9. Procédé pour produire des portions de viande façonnées selon l'une quelconque des revendications précédentes, **caractérisé en ce que**, dans le dispositif de façonnage avec une multitude de cavités de façonnage, une portion de viande congelée est introduite dans chaque cavité de façonnage et que, ensuite est produite une portion de viande façonnée par pression dans chaque cavité de façonnage, ou que, par application de pression et par expulsion dans chaque cavité de façonnage sont produites plusieurs portions de viande façonnées.

10. Procédé pour produire des portions de viande façonnées selon l'une quelconque des revendications précédentes, **caractérisé en ce que** pour obtenir une forme différente des portions de viande après un processus de cuisson, les portions de viande sélectionnées sont transformées avec une intensité différente, les morceaux de viande dans ce cas étant spécifiquement coupés de sorte que la cavité de façonnage présente des taux de déformation différents.

11. Procédé pour produire des portions de viande façonnées selon l'une quelconque des revendications précédentes, **caractérisé en ce qu'**après le façonnage, les portions de viande façonnées sont panées et/ou assaisonnées encore une fois et/ou sont cuites après les éventuelles autres étapes du procédé, notamment par cuisson, étuvage ou rôtissage.

12. Dispositif pour la production des portions de viande façonnées par
• un dispositif de tri pour trier la viande en morceaux dont le poids est inférieur au poids théorique, en morceaux dont le poids correspond à un poids théorique et en morceaux dont le poids est supérieur à un poids théorique,
• un dispositif de découpage étant en mesure de couper les morceaux de viande en surpoids de façon à atteindre le poids théorique, les morceaux de viande à poids précis et coupés ou les groupements de morceaux de viande à poids précis étant introduits comme morceaux de viande sélectionnés dans une ligne de traitement,
• un dispositif de transport pour continuer le transport des morceaux de viande sélectionnés,
• une unité de congélation par laquelle le dispositif de transport peut transporter les morceaux de viande sélectionnés et qui est destinée à congeler les morceaux de viande sélectionnés,
• un dispositif de façonnage pouvant presser les morceaux de viande sélectionnés congelés de manière contrôlée dans une cavité et étant en mesure de les transformer en forme finale ou plusieurs formes finales comme portions de viande avec poids et forme tridimensionnelles prédéterminés,
**caractérisé en ce que**
le dispositif est conçu pour la réalisation d'un procédé afin de produire des portions de viande façonnées selon l'une quelconque des revendications précédentes, le dispositif pour réaliser l'étape de préformation présentant des moyens pour préformer les morceaux de viande sélectionnés avant la congélation dans lesquels les morceaux de viande sélectionnés par préformation peuvent être introduits ou pressés et par lesquels les morceaux de viande sélectionnés préformés peuvent être congelés.

13. Dispositif pour produire des portions de viande façonnées selon l'une quelconque des revendications précédentes, **caractérisé en ce que** le moyen présente des moules de congélation pour la préformation qui font partie intégrante du dispositif de transport, notamment des cavités de moulage imprimées dans un convoyeur.

14. Dispositif pour produire des portions de viande façonnées selon l'une quelconque des deux revendications précédentes, **caractérisé en ce qu'**il présente un moyen pour la détermination du poids, ce dispositif pouvant calculer le poids notamment en raison d'un volume identifié et d'une valeur de densité prédéterminée.
